# EUROPEAN PATENT APPLICATION

(11) **EP 4 122 582 A1**
(43) Date of publication of application: **25.01.2023**
(21) Application number: 21788989.8
(22) Date of filing: 13.04.2021
(51) Int. Cl.: B01D 53/06, A61L 9/014

(54) **AIR TREATMENT DEVICE**

(30) Priority: 17.04.2020 JP 2020074125
(71) Applicant: Daikin Industries, Ltd., Osaka-shi, Osaka 530-0001 (JP)
(72) Inventor: KOIZUMI, Shin, Osaka-shi, Osaka 530-8323 (JP); TANAKA, Toshio, Osaka-shi, Osaka 530-8323 (JP); MOTEGI, Kanji, Osaka-shi, Osaka 530-8323 (JP)
(74) Representative: Hoffmann Eitle
(86) International application number: PCT/JP2021/015346
(87) International publication number: WO 2021/210584

(57) **Abstract**

An air treatment device (10) includes: a treater (P) configured to capture a toxic substance contained in target air; a regenerator (R) configured to remove the toxic substance from the treater (P); a detector (60) configured to detect an index correlated with a concentration of the toxic substance contained in room air; and a control unit (90) configured to control the regenerator (R) according to a detection value detected by the detector (60).

## Description

### TECHNICAL FIELD

The present disclosure relates to an air treatment device.

### BACKGROUND ART

An air purifier (air treatment device) that removes a toxic substance contained in room air has been known in the art. Patent Document 1 discloses an air purifier that purifies contaminated air containing a volatile organic compound (VOC) generated in a clean room of an electronic device manufacturing plant or any other similar space. The air purifier includes a rotor (treater) having a treatment zone and a desorption zone, and a heater.

Passing room air through the treatment zone in the air purifier causes the VOC contained in the room air to be adsorbed onto an adsorbent. Thus, the VOC is separated and removed from the room air. Passing outside air heated by a heater (heating section) through the desorption zone in the air purifier increases the temperature of the desorption zone. Thus, the VOC is desorbed from the adsorbent in the desorption zone. In this manner, the rotor is regenerated to be able to adsorb the VOC again.

### CITATION LIST

### PATENT DOCUMENTS

Patent Document 1: Japanese Unexamined Patent Publication No. 2017-51889

### SUMMARY OF THE INVENTION

### TECHNICAL PROBLEM

In a general air treatment device, air with a fixed temperature is supplied to a desorption zone, irrespective of the amount of a VOC adsorbed onto a treater. For this reason, a small amount of the VOC adsorbed increases the temperature of the treater to a higher temperature than necessary. This may cause excessive energy to be consumed to regenerate the treater.

It is an object of the present disclosure to reduce the energy consumed to regenerate a treater.

### SOLUTION TO THE PROBLEM

A first aspect of the present disclosure is directed to an air treatment device including: a treater (P) configured to capture a toxic substance contained in target air; a regenerator (R) configured to remove the toxic substance from the treater (P); a detector (60) configured to detect an index correlated with a concentration of the toxic substance contained in room air; and a control unit (90) configured to control the regenerator (R) according to a detection value detected by the detector (60).

According to the first aspect, the control unit (90) controls the regenerator (R) according to the detection value detected by the detector (60). This reduces the energy consumed to regenerate the treater (P).

A second aspect of the present disclosure is an embodiment of the first aspect. The air treatment device according to the second aspect further includes: a treatment-side passage (20) through which the target air flows; and a regeneration-side passage (30) through which regeneration air flows. The treater (P) is a treatment rotor (50) disposed to intersect the treatment-side passage (20) and the regeneration-side passage (30) and rotating, the regenerator (R) is a heating section (H) configured to heat the regeneration air to be supplied to the treater (P), and the control unit (90) adjusts an amount of the regeneration air heated by the heating section (H) according to the detection value detected by the detector (60).

According to the second aspect, the control unit (90) adjusts the amount of the regeneration air heated by the heating section (H) according to the detection value detected by the detector (60). This reduces the energy consumed to regenerate the treatment rotor (50).

A third aspect of the present disclosure is an embodiment of the second aspect. In the third aspect, the control unit (90) reduces the amount of the regeneration air heated by the heating section (H) as the concentration of the toxic substance contained in the room air decreases.

According to the third aspect, the lower the concentration of the toxic substance in the room air is, the smaller the amount of the heated regeneration air becomes.

A fourth aspect of the of the present disclosure is an embodiment of the first aspect. In the fourth aspect, the treater (P) includes an adsorbent that adsorbs the toxic substance, the regenerator (R) is a discharge mechanism (80) configured to discharge gas from the treater (P) to desorb the toxic substance from the adsorbent, the air treatment device (10) performs an adsorption operation of adsorbing the toxic substance in the target air onto the treater (P), and a regeneration operation of actuating the discharge mechanism (80) to desorb the toxic substance from the adsorbent in the treater (P), and the control unit (90) adjusts the number of actuations of the discharge mechanism (80) per predetermined time according to the detection value detected by the detector (60).

According to the fourth aspect, the control unit (90) adjusts the number of actuations of the discharge mechanism (80) per predetermined time according to the detection value detected by the detector (60). This can reduce the energy consumed to regenerate the treater (P).

A fifth aspect of the present disclosure is an embodiment of the fourth aspect. In the fifth aspect, the control unit (90) reduces the number of the actuations of the discharge mechanism (80) per predetermined time as the concentration of the toxic substance contained in the room air decreases.

According to the fifth aspect, the lower the concentration of a toxic substance in the room air is, the smaller the number of the actuations of the discharge mechanism (80) per predetermined time becomes.

A sixth aspect of the present disclosure is an embodiment of any one of the first to fifth aspects. In the sixth aspect, the treater (P) captures carbon dioxide, which is the toxic substance.

### BRIEF DESCRIPTION OF THE DRAWINGS

FIG. 1 illustrates a general configuration of an air treatment device according to a first embodiment.
FIG. 2 is a flowchart illustrating a control operation performed by a control unit according to the first embodiment.
FIG. 3 illustrates a general configuration of an air treatment device according to a second embodiment, and illustrates a state of the air treatment device that is performing a first operation.
FIG. 4 illustrates a state of the air treatment device that is performing a second operation, and corresponds to FIG. 3.
FIG. 5 is a block diagram illustrating relationship between a control unit according to the second embodiment and its peripheral devices.
FIG. 6 is a timing chart showing how first and second adsorption columns operate.

### DESCRIPTION OF EMBODIMENTS

### «First Embodiment»

An air treatment device (10) of a first embodiment will be described.

The air treatment device (10) of this embodiment removes a toxic substance contained in air in an indoor space (11) of a building or any other similar construction (hereinafter referred to as "room air (RA)") to purify the air, and supplies the air from which the toxic substance has been removed, as supply air (SA), to the indoor space (11).

The indoor space (11) has an air return port (11a) and an air supply port (11b). The air return port (11a) is used to supply the room air (RA) to the air treatment device (10). The air supply port (11b) is used to supply the supply air (SA) that has flowed out of the air treatment device (10) to the indoor space (11). In this embodiment, the toxic substance is carbon dioxide.

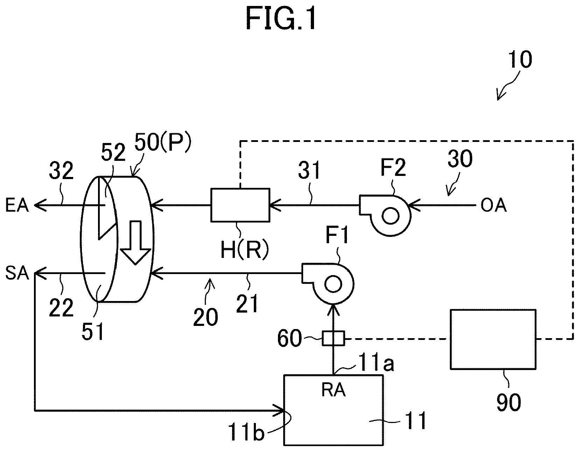

As illustrated in FIG. 1, the air treatment device (10) includes a treatment-side passage (20), a regeneration-side passage (30), a treatment rotor (50), a detector (60), and a control unit (90). The treatment rotor (50) is disposed to intersect the treatment-side passage (20) and the regeneration-side passage (30).

### <Treatment-side Passage>

The treatment-side passage (20) is used to remove carbon dioxide contained in the room air (RA) and to supply the purified air as the supply air (SA) to the indoor space (11). The treatment-side passage (20) allows target air flowing from the indoor space (11) thereinto to flow therethrough. The treatment-side passage (20) is configured such that the target air passes through the treatment rotor (50) only once.

The treatment-side passage (20) has a first treatment path (21) and a second treatment path (22). The first and second treatment paths (21) and (22) communicate with each other. The treatment-side passage (20) allows the target air to flow therethrough from the first treatment path (21) toward the second treatment path (22).

The first treatment path (21) is disposed upstream of the treatment rotor (50). An inlet end of the first treatment path (21) is connected to the air return port (11a) of the indoor space (11). The second treatment path (22) is disposed downstream of the treatment rotor (50). An outlet end of the second treatment path (22) is connected to the air supply port (11b) of the indoor space (11).

A first fan (F1) is disposed in the first treatment path (21). The first fan (F1) is used to send the room air (RA) to the treatment rotor (50).

### <Regeneration-side Passage>

The regeneration-side passage (30) is used to regenerate the treatment rotor (50) and to discharge air containing carbon dioxide as exhaust air (EA) to the outside of the room. The "regeneration" as used herein means that carbon dioxide (the toxic substance) is desorbed from the treatment rotor (50).

Regeneration air for regenerating the treatment rotor (50) flows through the regeneration-side passage (30). The target air and the regeneration air each flow from one surface toward the other surface of the treatment rotor (50) in the axial direction (the thickness direction). The regeneration-side passage (30) is configured such that the regeneration air passes through the treatment rotor (50) only once.

The regeneration-side passage (30) has a first regeneration path (31) and a second regeneration path (32). The first and second regeneration paths (31) and (32) communicate with each other. The regeneration-side passage (30) allows the regeneration air to flow therethrough from the first regeneration path (31) toward the second regeneration path (32).

The first regeneration path (31) is disposed upstream of the treatment rotor (50). An inlet end of the first regeneration path (31) communicates with the outside of the room. The second regeneration path (32) is disposed downstream of the treatment rotor (50). An outlet end of the second regeneration path (32) communicates with the outside of the room.

A second fan (F2) and a heater (H) are disposed in the first regeneration path (31). The second fan (F2) is used to send outdoor air (OA) to the treatment rotor (50). The second fan (F2) is disposed near the inlet end of the first regeneration path (31).

The heater (H) heats the regeneration air that is about to be supplied to the treatment rotor (50). The heater (H) is disposed downstream of the second fan (F2) in the first regeneration path (31). The heater (H) to which a predetermined current is supplied generates heat. The heater (H) corresponds to a heating section and a regenerator of the present disclosure.

### <Treatment Rotor>

The treatment rotor (50) captures the toxic substance contained in the target air. Specifically, the treatment rotor (50) captures and desorbs carbon dioxide. The treatment rotor (50) corresponds to a treater (P) of the present disclosure. The regenerator (R) eliminates the toxic substance from the treater (P).

The treatment rotor (50) is formed in the shape of a thick disk. The treatment rotor (50) is configured to enable passage of air therethrough in the thickness direction. The treatment rotor (50) includes a porous base material and a scavenger capturing carbon dioxide. Examples of the scavenger include an adsorbent, an absorbent, a collector, and a sorbent.

Passing the target air through the treatment rotor (50) allows the scavenger on the surface of the base material to capture carbon dioxide contained in the target air. Thus, carbon dioxide is removed from the target air. Passage of the regeneration air heated to a predetermined temperature through the treatment rotor (50) leads to desorption of carbon dioxide from the scavenger. Thus, the treatment rotor (50) is regenerated.

The base material of the treatment rotor (50) to be used is a material such as mesoporous silica, a porous polymer, or a resin. The scavenger of the treatment rotor (50) to be used is a substance capable of absorbing carbon dioxide, such as monoethanolamine (MEA), diethanolamine (DEA), or polyethyleneimine (PEI). Such a substance used as the scavenger has permeated the base material.

The treatment rotor (50) is provided to intersect the treatment-side passage (20) and the regeneration-side passage (30). The treatment rotor (50) is rotatable about its center axis. The treatment rotor (50) is driven to rotate by a motor (not shown), and moves continuously between the treatment-side passage (20) and the regeneration-side passage (30). The treatment rotor (50) is divided into a treatment zone (51) and a regeneration zone (52).

The treatment zone (51) and the regeneration zone (52) are fan-shaped portions concentric with the treatment rotor (50). The treatment zone (51) is a portion of the treatment rotor (50) that intersects the treatment-side passage (20). The regeneration zone (52) is a portion of the treatment rotor (50) that intersects the regeneration-side passage (30). The treatment zone (51) captures carbon dioxide contained in the target air. Passing the regeneration air heated by the heater (H) through the regeneration zone (52) leads to regeneration of the treatment rotor (50).

The air treatment device (10) makes the treatment zone (51) of the treatment rotor (50) capture carbon dioxide contained in the target air, and supplies the target air from which carbon dioxide has been removed by passing through the treatment zone (51), to the indoor space (11). The air treatment device (10) makes the regeneration air heated by the heater (H) pass through the regeneration zone (52) of the treatment rotor (50), and discharges the regeneration air containing carbon dioxide desorbed in the regeneration zone (52) to the outside of the room.

### <Detector>

The air treatment device (10) includes the detector (60). The detector (60) of this embodiment is a carbon dioxide sensor, and detects the concentration of carbon dioxide contained in the room air (RA). The concentration of carbon dioxide contained in the room air (RA) is an index correlated with the concentration of the toxic substance contained in the room air (RA). The detector (60) is disposed upstream of the first fan (F1) in the first treatment path (21). The detector (60) may be disposed in the indoor space (11).

### <Control Unit>

The control unit (90) controls the heating section (H) according to a detection value detected by the detector (60). Specifically, the control unit (90) operates the heater (H). The control unit (90) includes a microcomputer mounted on a control board, and a memory device (specifically, a semiconductor memory) storing software for operating the microcomputer.

The control unit (90) receives the detection value detected by the detector (60). The control unit (90) performs control according to the received detection value so that a predetermined current flows through the heater (H). The control unit (90) adjusts the amount of heat generated by the heater (H) by controlling the current flowing through the heater (H). Thus, the amount of the heated regeneration air is adjusted.

Specifically, the control unit (90) reduces the amount of heat generated by the heater (H) and reduces the amount of the heated regeneration air as the detection value detected by the detector (60) decreases (with a decreasing concentration of carbon dioxide). The control unit (90) increases the amount of heat generated by the heater (H) and increases the amount of the heated regeneration air as the detection value detected by the detector (60) increases (with an increasing concentration of carbon dioxide).

The control unit (90) controls the heater (H) using pulse width modulation (PWM). Specifically, the control unit (90) produces a fixed period of "on" and "off" times during a pulse train, from the input of a fixed current to the heater (H), and varies the width of the "on" time (duty cycle D). The duty cycle D is expressed as the ratio of the width W of the "on" time to a pulse period T. In other words, it is expressed as D = (W/T) × 100 [%].

The control unit (90) generates a PWM signal (duty cycle D), and allows current to flow through the heater (H) only for the "on" time during one pulse period based on the generated PWM signal. The higher the duty cycle D is, the larger the amount of heat generated by the heater (H) is.

### -Operation of Air Treatment Device-

Next, how the air treatment device (10) operates will be described. The start of operation of the air treatment device (10) causes the treatment rotor (50) to be driven to rotate, and causes the first and second fans (F1) and (F2) to work.

The working first fan (F1) causes the room air (RA) to flow from the indoor space (11) into the first treatment path (21). The room air (RA) that has flowed into the first treatment path (21) passes through the first fan (F1), and flows into the treatment zone (51) of the treatment rotor (50). In the treatment zone (51), carbon dioxide in the target air is captured by the treatment rotor (50).

The target air from which carbon dioxide has been removed passes through the second treatment path (22), and is supplied as the supply air (SA) to the indoor space (11). Thus, carbon dioxide in the indoor space (11) is removed. A portion of the air in the indoor space (11) again flows through the air return port (11a) into the first treatment path (21).

The working second fan (F2) causes the outdoor air (OA) to flow from the outside of the room into the first regeneration path (31). The outdoor air (OA) that has flowed into the first regeneration path (31) passes, as regeneration air, through the second fan (F2), and flows into the heater (H). The regeneration air that has flowed into the heater (H) is heated while passing through the heater (H).

The heated regeneration air flows into the regeneration zone (52) of the treatment rotor (50). While the regeneration air passes through the regeneration zone (52), carbon dioxide captured by the scavenger is desorbed into the regeneration air in the regeneration zone (52). The regeneration air that has passed through the regeneration zone (52) is discharged, as the exhaust air (EA), through the second regeneration path (32) to the outside of the room.

### -Control Operation of Control Unit-

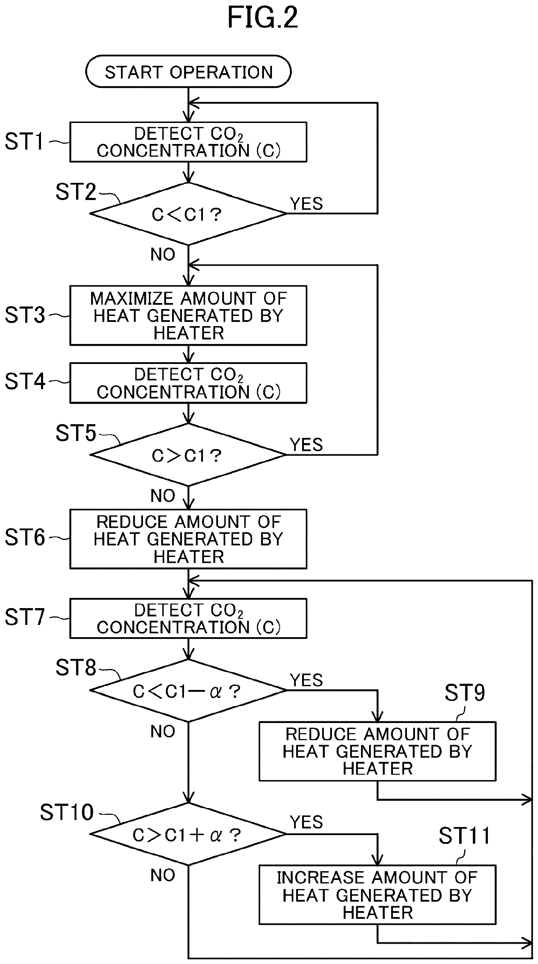

An operation in which the control unit (90) controls the heater (H) will be described with reference to the flowchart of FIG. 2. The control unit (90) performs this operation while the air treatment device (10) is in operation.

### <Step ST1>

When operation of the air treatment device (10) is started, the control unit (90) performs the process of step ST1. In the process of step ST1, the control unit (90) reads a detection value (in this embodiment, the carbon dioxide concentration in the room) C detected by the detector (60).

### <Step ST2>

Next, the control unit (90) performs the process of step ST2. In the process of step ST2, the control unit (90) compares the carbon dioxide concentration C in the room with a predetermined reference value C1. In this embodiment, the value C1 is 1000 ppm.

If the carbon dioxide concentration C in the room is lower than the predetermined reference value C1 (C < C1), the control unit (90) performs the process of step ST1. On the other hand, if the carbon dioxide concentration C in the room is higher than or equal to the predetermined reference value C1 (C ≥ C1), the control unit (90) performs the process of step ST3.

### <Step ST3>

In the process of step ST3, the control unit (90) passes a current through the heater (H) such that the amount of heat generated by the heater (H) is maximum. Specifically, the control unit (90) continues energizing the heater (H) with the duty cycle D of the PWM signal set to 100.

Since the carbon dioxide concentration C in the room is higher than or equal to the predetermined reference value C1 (C ≥ C1), the amount of heat generated by the heater (H) is maximized to rapidly lower the carbon dioxide concentration in the room.

### <Step ST4>

Next, the control unit (90) performs the process of step ST4. In the process of step ST4, the control unit (90) again reads the carbon dioxide concentration C in the indoor space.

### <Step ST5>

Next, the control unit (90) performs the process of step ST5. In the process of step ST5, the control unit (90) compares the carbon dioxide concentration C in the room with the predetermined reference value C 1.

If the carbon dioxide concentration C is higher than the predetermined reference value C1 (C > C1), the control unit (90) performs the process of step ST3. In other words, the amount of heat generated by the heater (H) is kept maximum. On the other hand, if the carbon dioxide concentration C in the room is lower than or equal to the predetermined reference value C1 (C ≤ C1), the control unit (90) performs the process of step ST6.

### <Step ST6>

In the process of step ST6, the control unit (90) passes a current through the heater (H) such that the amount of heat generated by the heater (H) decreases. Specifically, the control unit (90) reduces the duty cycle D of the PWM signal, thereby reducing the "on" time during one pulse period.

### <Step ST7>

Next, the control unit (90) performs the process of step ST7. In the process of step ST7, the control unit (90) again reads the carbon dioxide concentration C in the indoor space.

### <Step ST8>

Next, the control unit (90) performs the process of step ST8. In the process of step ST8, the control unit (90) compares the carbon dioxide concentration C in the room with the predetermined reference value C1 - α. In this embodiment, the value α is 200 ppm. The value α may be greater than or equal to 200 ppm (e.g., 500 ppm).

If the carbon dioxide concentration C in the room is lower than the predetermined reference value C1 - α (C < C1 - α), the control unit (90) performs the process of step ST9. On the other hand, if the carbon dioxide concentration C in the room is higher than or equal to the predetermined reference value C1 - α (C ≥ C1 - α), the control unit (90) performs the process of step ST10.

### <Step ST9>

In the process of step ST9, the control unit (90) passes a current through the heater (H) such that the amount of heat generated by the heater (H) decreases. Specifically, the control unit (90) reduces the duty cycle D of the PWM signal, thereby reducing the "on" time during one pulse period. Thereafter, the control unit (90) performs the process of step ST7.

### <Step ST10>

In the process of step ST10, the control unit (90) compares the carbon dioxide concentration C in the room with the predetermined reference value C1 + α.

If the carbon dioxide concentration C in the room is higher than the predetermined reference value C1 + α (C > C1 + α), the control unit (90) performs the process of step ST11. On the other hand, if the carbon dioxide concentration C in the room is lower than or equal to the predetermined reference value C1 + α (C ≤ C1 + α), the control unit (90) performs the process of step ST7.

### <Step ST11>

In the process of step ST11, the control unit (90) passes a current through the heater (H) such that the amount of heat generated by the heater (H) increases. Specifically, the control unit (90) increases the duty cycle D of the PWM signal, thereby increasing the "on" time during one pulse period. Thereafter, the control unit (90) performs the process of step ST7.

As can be seen, if the carbon dioxide concentration C in the room is low, the amount of heat generated by the heater (H) is reduced, and if the carbon dioxide concentration C in the room is high, the amount of heat generated by the heater (H) is increased. This enables heating of the regeneration air to an appropriate temperature. As a result, the energy consumed to regenerate the treatment rotor (50) can be reduced.

### -Feature (1) of First Embodiment-

The air treatment device (10) of this embodiment includes the detector (60) that detects the index correlated with the concentration of the toxic substance contained in the room air (RA), and the control unit (90) that controls the regenerator (R) according to the detection value detected by the detector (60).

In the air treatment device (10) of this embodiment, the control unit (90) controls the regenerator (R) according to the detection value detected by the detector (60). This can reduce the energy consumed to regenerate the treater (P).

### -Feature (2) of First Embodiment-

The air treatment device (10) of this embodiment includes the treater (P) corresponding to the treatment rotor (50), and the regenerator (R) corresponding to the heater (H). The control unit (90) adjusts the amount of the regeneration air heated by the heater (H) according to the detection value detected by the detector (60).

In a general air treatment device, air with a fixed temperature is supplied to the treatment zone (51), irrespective of the amount of carbon dioxide captured by the treatment rotor (50). For this reason, a small amount of carbon dioxide treated increases the temperature of the treatment rotor (50) to a higher temperature than necessary. This may cause excessive energy to be consumed to regenerate the treatment rotor (50).

In the air treatment device (10) of this embodiment, the amount of the heated regeneration air is adjusted according to the detection value detected by the detector (60). This can substantially prevent the temperature of the treatment rotor (50) from increasing to a higher temperature than necessary. This can reduce the energy consumed to regenerate the treatment rotor (50).

### -Feature (3) of First Embodiment-

The heater (H) of the air treatment device (10) of this embodiment reduces the amount of the heated regeneration air as the carbon dioxide concentration decreases.

In the air treatment device (10) of this embodiment, the lower carbon dioxide concentration in the room air (RA) is, the smaller the amount of the heated regeneration air becomes. This can reduce the energy consumed to regenerate the treatment rotor (50).

### «Second Embodiment»

An air treatment device (10) of a second embodiment will be described. The air treatment device (10) of this embodiment is a modified version, of the air treatment device (10) of the first embodiment, in which the configurations of the treater (P) and the regenerator (R) have been changed. Thus, the following description will be focused on the differences between the air treatment device (10) of this embodiment and the air treatment device (10) of the first embodiment.

Just like the first embodiment, the air treatment device (10) of this embodiment removes a toxic substance contained in room air (RA) to purify the room air (RA), and supplies the air from which the toxic substance has been removed, as supply air (SA), to an indoor space (11). The air treatment device (10) of this embodiment includes adsorption columns (47, 48) instead of the treatment rotor (50) of the first embodiment, as the treater (P), and includes a suction pump (80) instead of the heating section (H) of the first embodiment, as the regenerator (R). In the air treatment device (10) of this embodiment, the pressure of an adsorbent in each adsorption column (47, 48) is reduced by the suction pump (80), thereby desorbing carbon dioxide. The suction pump (80) corresponds to a discharge mechanism of the present disclosure.

Specifically, as illustrated in FIG. 1, the air treatment device (10) includes a first adsorption column (47), a second adsorption column (48), first to sixth on-off valves (41, 42, 43, 44, 45, 46), a fan (F), the suction pump (80), a detector (60), and a control unit (90). The air treatment device (10) includes a first treatment path (21), a second treatment path (22), a suction passage (81), a first relay path (71), and a second relay path (72).

### <Adsorption Column>

Each of the first and second adsorption columns (47) and (48) is a member including a cylindrical container with both ends closed and an adsorbent that fills the container. The adsorbent that fills these adsorption columns (47, 48) has the property of adsorbing a carbon dioxide component, and the property of desorbing the adsorbed carbon dioxide component by reducing the pressure of the adsorbent that has adsorbed the carbon dioxide component. The first and second adsorption columns (47) and (48) correspond to the treater (P) of the present disclosure.

### <First Relay Path>

The first relay path (71) has an outlet end connected to the first adsorption column (47). The first relay path (71) branches into two passages near its inlet end. One of the branch passages is connected to the first on-off valve (41). The other branch passage is connected to the third on-off valve (43).

### <Second Relay Path>

The second relay path (72) has an outlet end connected to the second adsorption column (48). The second relay path (72) branches into two passages near its inlet end. One of these branch passages is connected to the second on-off valve (42). The other branch passage is connected to the fourth on-off valve (44).

### <First Treatment Path>

The first treatment path (21) is disposed upstream of the first and second adsorption columns (47) and (48). An inlet end of the first treatment path (21) is connected to an air return port (11a) of the indoor space (11). The first treatment path (21) branches into two passages near its outlet end. One of these branch passages is connected to the first on-off valve (41). The other branch passage is connected to the second on-off valve (42).

### <Fan>

The fan (F) is disposed in the first treatment path (21). Specifically, the fan (F) is disposed upstream of the branch point of the first treatment path (21). The fan (F) sends room air (RA) to the first adsorption column (47) or the second adsorption column (48). The fan (F) may be a suction pump. The fan (F) may be disposed downstream of the adsorption columns (47, 48) (in the second treatment path (22)).

### <Second Treatment Path>

The second treatment path (22) is disposed downstream of the first and second adsorption columns (47) and (48). An outlet end of the second treatment path (22) is connected to an air supply port (11b) of the indoor space (11). The second treatment path (22) branches into two passages near its inlet end. One of these branch passages is connected to the first adsorption column (47). The other branch passage is connected to the second adsorption column (48).

The fifth and sixth on-off valves (45) and (46) are disposed upstream of the branch point of the second treatment path (22). Specifically, the fifth on-off valve (45) is disposed between the branch point of the second treatment path (22) and the first adsorption column (47). The sixth on-off valve (46) is disposed between the branch point of the second treatment path (22) and the second adsorption column (48).

### <Suction Passage>

The suction passage (81) branches into two passages near its inlet end. One of these branch passages is connected to the third on-off valve (43). The other branch passage is connected to the fourth on-off valve (44). An outlet end of the suction passage (81) communicates with the outside of the air treatment device (10).

### <Suction Pump>

The suction pump (80) is disposed in the suction passage (81). Specifically, the suction pump (80) is disposed downstream of the branch point of the suction passage (81). The suction pump (80) sucks gas from the first adsorption column (47) or the second adsorption column (48) to desorb carbon dioxide from the adsorbent. The suction pump (80) corresponds to the regenerator (R) of the present disclosure.

### <On-Off Valve>

Each of the first to sixth on-off valves (41, 42, 43, 44, 45, 46) is configured as an electromagnetic valve. The first on-off valve (41) is opened when target air flowing through the first treatment path (21) is to be sent to the first adsorption column (47). The second on-off valve (42) is opened when the target air flowing through the first treatment path (21) is to be sent to the second adsorption column (48).

The third on-off valve (43) is opened when exhaust air (EA) containing carbon dioxide desorbed from the adsorbent in the first adsorption column (47) is to be discharged to the outside of the room. The fourth on-off valve (44) is opened when exhaust air (EA) containing carbon dioxide desorbed from the adsorbent in the second adsorption column (48) is to be discharged to the outside of the room.

The fifth on-off valve (45) is opened when treated air treated in the first adsorption column (47) is to be sent, as supply air (SA), to the indoor space (11). The sixth on-off valve (46) is opened when treated air treated in the second adsorption column (48) is to be sent, as supply air (SA), to the indoor space (11).

The air treatment device (10) of this embodiment may be provided with switching valves each having three ports instead of the first and third on-off valves (41) and (43), and may be provided with switching valves each having three ports instead of the second and fourth on-off valves (42) and (44), just like the foregoing description.

### <Control Unit>

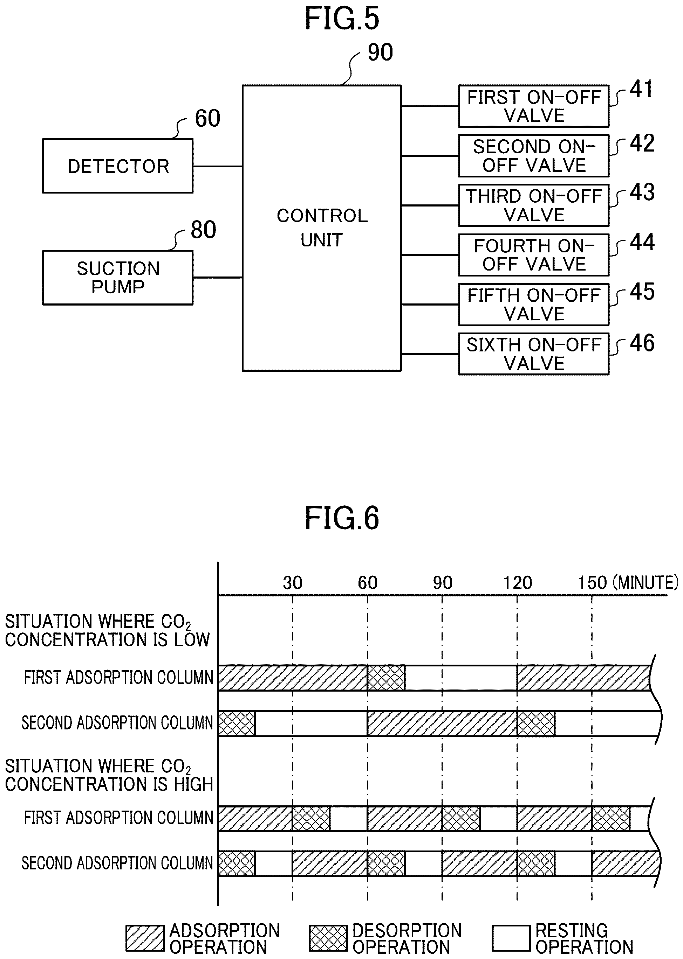

The control unit (90) illustrated in FIG. 5 is connected to the detector (60), the first to sixth on-off valves (41, 42, 43, 44, 45, 46), and the suction pump (80) via wires. Signals are exchanged between these components and the control unit (90).

The control unit (90) receives the detection value detected by the detector (60). The control unit (90) controls the first to sixth on-off valves (41, 42, 43, 44, 45, 46) and the suction pump (80) according to a detection value detected by the detector (60). Specifically, the control unit (90) controls the opening and closing of the first to sixth on-off valves (41, 42, 43, 44, 45, 46) to change the state of communication among the passages. The control unit (90) adjusts the number of actuations of the suction pump (80) per predetermined time according to the detection value detected by the detector (60). The control unit (90) reduces the number of actuations of the suction pump (80) per predetermined time as the concentration of carbon dioxide contained in the room air (RA) decreases.

### - Operation of Air Treatment Device-

Next, how the air treatment device (10) of this embodiment operates will be described.

The air treatment device (10) of this embodiment alternately and repeatedly performs a first operation and a second operation to be described later for a predetermined time each, thereby removing carbon dioxide from the room air (RA) and supplying the air from which carbon dioxide has been removed to the indoor space (11).

### <First Operation>

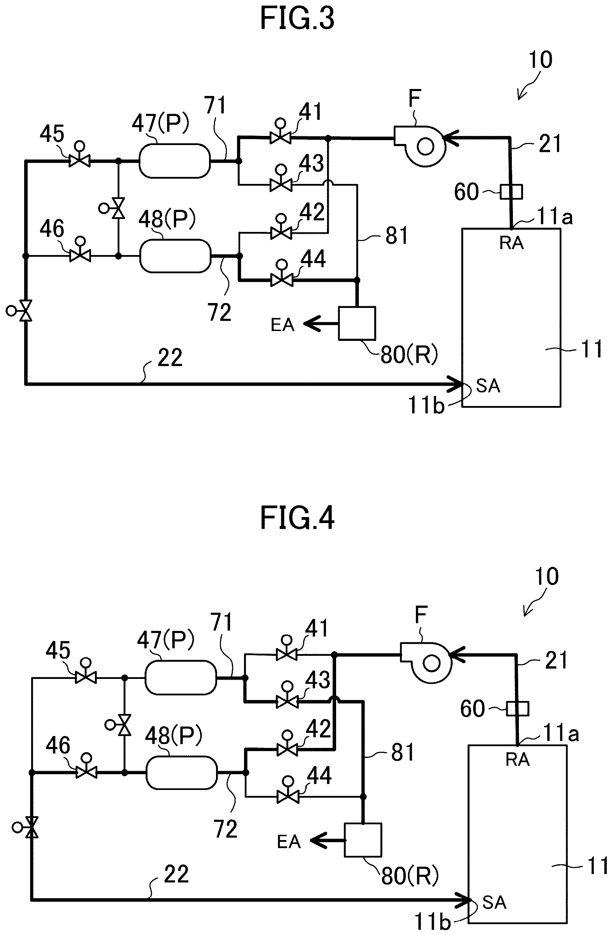

As illustrated in FIG. 3, in the first operation, the first on-off valve (41) is opened, and the second on-off valve (42) is closed, thereby making the air return port (11a) of the indoor space (11) communicate with the first adsorption column (47). In the first operation, the fifth on-off valve (45) is opened, and the sixth on-off valve (46) is closed, thereby making the first adsorption column (47) communicate with the air supply port (11b) of the indoor space (11). In the first operation, the third on-off valve (43) is closed, and the fourth on-off valve (44) is opened, thereby making the second adsorption column (48) communicate with the suction pump (80).

In the first operation, an adsorption operation intended for the first adsorption column (47) and a regeneration operation and a resting operation both intended for the second adsorption column (48) are performed. In the first operation, while the adsorption operation is being performed in the first adsorption column (47), the regeneration operation is performed in the second adsorption column (48), and then the resting operation is performed.

In the adsorption operation, the fan (F) supplies target air through the first treatment path (21) and the first relay path (71) to the first adsorption column (47). In the first adsorption column (47), carbon dioxide contained in the supplied target air is adsorbed by the adsorbent. As a result, in the first adsorption column (47), treated air having a lower carbon dioxide concentration than the target air is produced. The produced treated air flows out of the first adsorption column (47), flows through the second treatment path (22), and is supplied, as supply air (SA), to the indoor space (11). The adsorption operation is executed over a predetermined first time.

In the regeneration operation, the suction pump (80) is actuated to suck air from the second adsorption column (48). The internal pressure of the second adsorption column (48) decreases to desorb carbon dioxide from the adsorbent. As a result, in the second adsorption column (48), exhaust air (EA) having a higher carbon dioxide concentration than the target air is produced. The produced exhaust air (EA) flows from the second adsorption column (48) into the second relay path (72) and the suction passage (81), and is sucked into the suction pump (80). The suction pump (80) discharges the sucked exhaust air (EA) to the outside of the room. The regeneration operation is performed over a predetermined second time. When the duration of the regeneration operation reaches the second time, the suction pump (80) stops.

The resting operation is performed for the second adsorption column (48) that has finished undergoing the regeneration operation. In the resting operation, the sixth on-off valve (46) is opened with the suction pump (80) at rest. Opening the sixth on-off valve (46) allows the second adsorption column (48) to have a pressure equal to that of the supply air (SA). The second adsorption column (48) having a pressure equal to that of the supply air (SA) causes the flow of air in the suction passage (81) to stop.

### <Second Operation>

As illustrated in FIG. 4, in the second operation, the second on-off valve (42) is opened, and the first on-off valve (41) is closed, thereby making the air return port (11a) of the indoor space (11) communicate with the second adsorption column (48). In the second operation, the sixth on-off valve (46) is opened, and the fifth on-off valve (45) is closed, thereby making the second adsorption column (48) communicate with the air supply port (11b) of the indoor space (11). In the second operation, the fourth on-off valve (44) is closed, and the third on-off valve (43) is opened, thereby making the first adsorption column (47) communicate with the suction pump (80).

In the second operation, an adsorption operation intended for the second adsorption column (48) and a regeneration operation and a resting operation both intended for the first adsorption column (47) are performed. In the second operation, while the adsorption operation is being performed in the second adsorption column (48), the regeneration operation is performed in the first adsorption column (47), and then the resting operation is performed.

In the adsorption operation, the fan (F) supplies target air through the first treatment path (21) and the second relay path (72) to the second adsorption column (48). In the second adsorption column (48), carbon dioxide contained in the supplied target air is adsorbed by the adsorbent. As a result, in the second adsorption column (48), treated air having a lower carbon dioxide concentration than the target air is produced. The produced treated air flows out of the second adsorption column (48), flows through the second treatment path (22), and is supplied, as supply air (SA), to the indoor space (11). The adsorption operation is executed over the predetermined first time.

In the regeneration operation, the suction pump (80) is actuated to suck air from the first adsorption column (47). The internal pressure of the first adsorption column (47) decreases to desorb carbon dioxide from the adsorbent. As a result, in the first adsorption column (47), exhaust air (EA) having a higher carbon dioxide concentration than the target air is produced. The produced exhaust air (EA) flows from the first adsorption column (47) into the first relay path (71) and the suction passage (81), and is sucked into the suction pump (80). The suction pump (80) discharges the sucked exhaust air (EA) to the outside of the room. The regeneration operation is performed over the predetermined second time. When the duration of the regeneration operation reaches the second time, the suction pump (80) stops.

The resting operation is performed for the first adsorption column (47) that has finished undergoing the regeneration operation. In the resting operation, the fifth on-off valve (45) is opened with the suction pump (80) at rest. Opening the fifth on-off valve (45) allows the first adsorption column (47) to have a pressure equal to that of the supply air (SA). The first adsorption column (47) having a pressure equal to that of the supply air (SA) causes the flow of air in the suction passage (81) to stop.

Here, the predetermined first time during which the adsorption operation intended for the first adsorption column (47) in the first operation or the adsorption operation intended for the second adsorption column (48) in the second operation is performed is adjusted according to the carbon dioxide concentration detected by the detector (60). The predetermined second time during which the regeneration operation intended for the first adsorption column (48) in the first operation or the regeneration operation intended for the second adsorption column (47) in the second operation is performed is fixed irrespective of the carbon dioxide concentration detected by the detector (60). The second time is shorter than the first time.

### - Control Operation of Suction Pump-

Next, an operation in which the control unit (90) controls the suction pump (80) will be described with reference to FIG. 6.

The start of operation of the air treatment device (10) causes the control unit (90) to receive the carbon dioxide concentration in the room air (RA), as the detection value, from the detector (60). When the control unit (90) receives the detection value, the air treatment device (10) initially performs the first operation. The air treatment device (10) may start from the second operation.

Suppose here that, for example, carbon dioxide is desorbed from the adsorbent in each of the first and second adsorption columns (47) and (48) in 15 minutes. Suppose that if the carbon dioxide concentration input to the control unit (90) is low, it takes 60 minutes until the adsorbent in each of the first and second adsorption columns (47) and (48) becomes substantially incapable of adsorbing carbon dioxide. In other words, in this case, the first operation and the second operation are performed for 60 minutes each.

If the carbon dioxide concentration input to the control unit (90) is low, the first operation is performed over 60 minutes. In this first operation, the adsorption operation intended for the first adsorption column (47) is performed for 60 minutes. In this first operation, a regeneration operation intended for the second adsorption column (48) is performed for 15 minutes, and then the resting operation intended for the second adsorption column (48) is performed for 45 minutes.

After the first operation has ended, the second operation is performed over 60 minutes. In this second operation, the adsorption operation intended for the second adsorption column (48) is performed for 60 minutes. In this second operation, the regeneration operation intended for the first adsorption column (47) is performed for 15 minutes, and then the resting operation intended for the first adsorption column (47) is performed for 45 minutes. In other words, the control unit (90) actuates the suction pump (80) twice in 120 minutes during which the first and second operations are performed.

Suppose that on the other hand, if the carbon dioxide concentration input to the control unit (90) is high, it takes 30 minutes until the adsorbent in each of the first and second adsorption columns (47) and (48) becomes substantially incapable of adsorbing carbon dioxide. In other words, in this case, the first operation and the second operation are performed for 30 minutes each.

If the carbon dioxide concentration input to the control unit (90) is high, the first operation is performed over 30 minutes. In this first operation, the adsorption operation intended for the first adsorption column (47) is performed for 30 minutes. In this first operation, a regeneration operation intended for the second adsorption column (48) is performed for 15 minutes, and then the resting operation intended for the second adsorption column (48) is performed for 15 minutes.

After the first operation has ended, the second operation is performed over 30 minutes. In this second operation, the adsorption operation intended for the second adsorption column (48) is performed for 30 minutes. In this second operation, the desorption operation intended for the first adsorption column (47) is performed for 15 minutes, and then the resting operation intended for the first adsorption column (47) is performed for 15 minutes. In other words, the control unit (90) actuates the suction pump (80) four times in 120 minutes during which the first and second operations are performed.

As can be seen, if the carbon dioxide concentration in the indoor space (11) is low, the control unit (90) reduces the number of actuations of the suction pump (80) per predetermined time, and if the carbon dioxide concentration in the indoor space (11) is high, the control unit (90) increases the number of actuations of the suction pump (80) per predetermined time. Thus, the lower the carbon dioxide concentration in the indoor space (11) is, the smaller the number of actuations of the suction pump (80) per predetermined period becomes. In other words, the lower the carbon dioxide concentration in the indoor space (11) is, the longer the time during which the suction pump (80) is at rest per predetermined time becomes. As a result, the energy consumed to regenerate the first and second adsorption columns (47) and (48) can be reduced.

### - Feature (1) of Second Embodiment-

In the air treatment device (10) of this embodiment, the treater (P) corresponds to the first and second adsorption columns (47) and (48) each including the adsorbent, and the regenerator (R) corresponds to the suction pump (80). The control unit (90) adjusts the number of actuations of the suction pump (80) per predetermined time according to the detection value detected by the detector (60).

In the air treatment device (10) of this embodiment, the control unit (90) adjusts the number of actuations of the suction pump (80) per predetermined time according to the detection value detected by the detector (60). This can reduce the energy consumed to regenerate the first and second adsorption columns (47) and (48).

### - Feature (2) of Second Embodiment -

The control unit (90) of the air treatment device (10) of this embodiment reduces the number of actuations of the suction pump (80) per predetermined time as the concentration of the toxic substance contained in the room air decreases.

In the air treatment device (10) of this embodiment, the lower the concentration of a toxic substance contained in the room air (RA) is, the smaller the number of actuations of the suction pump per predetermined time becomes. In other words, the lower the concentration of the toxic substance in the room air (RA) is, the longer the time during which the suction pump (80) is at rest per predetermined time becomes. This can reduce the energy consumed to regenerate the first and second adsorption columns (47) and (48).

### <<Other Embodiments>>

The above-described embodiments may be modified as follows.

In the air treatment device (10) of each of the above-described embodiments, a target toxic substance may be a substance except carbon dioxide. The target toxic substance may be, for example, a VOC or formaldehyde.

While the target air of the first embodiment flows from one surface toward the other surface of the treatment rotor (50) in the axial direction, the regeneration air may flow from the other surface toward the one surface of the treatment rotor (50) in the axial direction.

The treatment rotor (50) of the first embodiment may include a purge zone. The purge zone is provided between the treatment zone (51) and the regeneration zone (52), and is a portion for cooling the regeneration zone (52) warmed by passage of air heated by the heater (H) through the regeneration zone (52).

The detector (60) of each of the above-described embodiments may be a sensor configured to detect the number of people in the indoor space (11). In this case, the detector (60) is disposed in the indoor space (11). Furthermore, in this case, the index correlated with the concentration of the toxic substance contained in the room air (RA) is the number of people in the indoor space (11).

The control unit (90) of the first embodiment may perform control to continue passing current through the heater (H) and continuously change the magnitude of the current.

While the embodiments and variations thereof have been described above, it will be understood that various changes in form and details may be made without departing from the spirit and scope of the claims. The embodiments, the variations, and the other embodiments may be combined and replaced with each other without deteriorating intended functions of the present disclosure. The ordinal numbers such as "first," "second," "third," ..., described above are used to distinguish the terms to which these expressions are given, and do not limit the number and order of the terms.

### INDUSTRIAL APPLICABILITY

As can be seen from the foregoing description, the present disclosure is useful for an air treatment device.

### DESCRIPTION OF REFERENCE CHARACTERS

- 10: Air Treatment Device
- 20: Treatment-side Passage
- 30: Regeneration-side Passage
- 50: Treatment Rotor
- 51: Treatment Zone
- 52: Regeneration Zone
- 60: Detector
- 80: Suction Pump (Discharge Mechanism)
- 90: Control Unit
- H: Heater (Heating Section)
- P: Treater
- R: Regenerator

## Claims

1. An air treatment device, comprising:
a treater (P) configured to capture a toxic substance contained in target air;
a regenerator (R) configured to remove the toxic substance from the treater (P);
a detector (60) configured to detect an index correlated with a concentration of the toxic substance contained in room air; and
a control unit (90) configured to control the regenerator (R) according to a detection value detected by the detector (60).

2. The air treatment device of claim 1, further comprising:
a treatment-side passage (20) through which the target air flows; and
a regeneration-side passage (30) through which regeneration air flows, wherein
the treater (P) is a treatment rotor (50) disposed to intersect the treatment-side passage (20) and the regeneration-side passage (30) and rotating,
the regenerator (R) is a heating section (H) configured to heat the regeneration air to be supplied to the treater (P), and
the control unit (90) adjusts an amount of the regeneration air heated by the heating section (H) according to the detection value detected by the detector (60).

3. The air treatment device of claim 2, wherein
the control unit (90) reduces the amount of the regeneration air heated by the heating section (H) as the concentration of the toxic substance contained in the room air decreases.

4. The air treatment device of claim 1, wherein
the treater (P) includes an adsorbent that adsorbs the toxic substance,
the regenerator (R) is a discharge mechanism (80) configured to discharge gas from the treater (P) to desorb the toxic substance from the adsorbent,
the air treatment device (10) performs an adsorption operation of adsorbing the toxic substance in the target air onto the treater (P), and a regeneration operation of actuating the discharge mechanism (80) to desorb the toxic substance from the adsorbent in the treater (P), and
the control unit (90) adjusts the number of actuations of the discharge mechanism (80) per predetermined time according to the detection value detected by the detector (60).

5. The air treatment device of claim 4, wherein
the control unit (90) reduces the number of the actuations of the discharge mechanism (80) per predetermined time as the concentration of the toxic substance contained in the room air decreases.

6. The air treatment device of any one of claims 1 to 5, wherein
the treater (P) captures carbon dioxide, which is the toxic substance.
